# FASCICULE DE BREVET EUROPEEN

(11) **EP 4 412 553 B1**
(45) Date de publication et mention de la délivrance du brevet: **25.12.2024**
(21) Numéro de dépôt: 23782901.5
(22) Date de dépôt: 29.09.2023
(51) Int. Cl.: A61B 50/13, A61B 90/50, B62B 5/04

(54) **STABILISATEUR POUR CHARIOTS MÉDICAUX**
STABILISATOR FÜR MEDIZINWAGEN
STABILISER FOR MEDICAL TROLLEYS

(30) Priorité: 29.12.2022 FR 2214665
(43) Date de publication de la demande: 14.08.2024
(73) Titulaire: Digicuto, 13008 Marseille (FR)
(72) Inventeur: KOUBI, Stefan, 13008 Marseille (FR); GUREL, Galip, 13008 Marseille (FR); TOURBAH, Karim, 13008 Marseille (FR)
(74) Mandataire: A.P.I. Conseil
(86) Numéro de dépôt international: PCT/EP2023/077078
(87) Numéro de publication internationale: WO 2024/141188

(56) Documents cités:
- US-A1- 2017 065 354
- US-A1- 2018 346 008
- US-B2- 10 123 842

## Description

### Domaine technique

L'invention concerne le domaine des systèmes de chariots médicaux. En particulier, elle concerne un dispositif de stabilisation pour chariots médicaux ainsi qu'une station mobile itinérante robotisée de traitement dentaire ou médical.

Des dispositifs similaires sont connus des documents US2018346008A1 et US2017065354A1.

### Technique antérieure

Dans la vie courante, les problèmes dentaires et médicaux sont malheureusement courants.

Par ailleurs, si les patients ne peuvent pas recevoir de traitements appropriés dans les temps, ces problèmes dentaires et médicaux peuvent être extrêmement graves et douloureux, affectant ainsi leur qualité de vie et leur santé.

Afin de traiter plus rapidement les patients, on envisage de plus en plus d'utiliser des robots pour assister les praticiens dans la réalisation des procédures dentaires ou médicales.

En effet, grâce à leurs mouvements contrôlables, les robots peuvent non seulement améliorer l'efficacité des procédures dentaires ou médicales, mais aussi l'efficacité du traitement en évitant ou en limitant les erreurs humaines.

Certains de ces robots sont prévus pour être montés sur des chariots médicaux de type connu et qui sont adaptés pour circuler sur le sol.

Or, le sol sur lequel peut circuler un chariot médical ne présente pas nécessairement une surface plane, de sorte que, lorsque le robot est en opération, il existe un risque quant au niveau de précision des mouvements du robot, ce qui implique un risque élevé pour la sécurité des patients.

Ainsi, il existe un besoin pour un dispositif de stabilisation pour chariots médicaux.

### Résumé de l'invention

L'invention vise à résoudre, au moins partiellement, ce besoin.

Un premier aspect de l'invention vise un dispositif de stabilisation pour un chariot médical comprenant un châssis de plancher porteur.

Le dispositif de stabilisation comprend,
- des pieds de stabilisation au sol qui sont configurés pour supporter le châssis de plancher porteur et qui sont convertibles entre une configuration déployée dans laquelle les pieds de stabilisation au sol sont en appui sur le sol, de façon stable, de manière à supporter, ensemble, tout ou partie de ce que le châssis de plancher porteur supporte, et une configuration repliée dans laquelle les pieds de stabilisation au sol sont décollés du sol de manière à assurer un escamotage au moins partiel,
- un mécanisme de commande qui est déplaçable entre une première position et une deuxième position, en réponse à au moins un mouvement de translation ou un mouvement de rotation,
- un mécanisme de transmission de commande qui comprend,
   - un ensemble de liaison, et
   - au moins un élément de transmission de commande qui présente une première portion dudit élément de transmission de commande reliée cinématiquement, de manière directe ou indirecte, au mécanisme de commande, et une deuxième portion dudit élément de transmission de commande reliée cinématiquement, de manière articulée, aux pieds de stabilisation au sol via l'ensemble de liaison, l'élément de transmission de commande étant monté rotatif autour d'un premier axe de rotation de manière à être entraîné en rotation dans un premier sens de rotation et dans un deuxième sens de rotation opposé au premier sens de rotation,
ledit ensemble de liaison comprenant,
- au moins une première articulation, à au moins un degré de liberté, qui est directement reliée à la deuxième portion dudit élément de transmission de commande et à au moins un premier pied de stabilisation au sol,
- au moins une deuxième articulation, à au moins un degré de liberté, qui est directement reliée à au moins un deuxième pied de stabilisation au sol, et
- une structure de liaison qui est reliée entre la première acticulation et la deuxième articulation.

En outre, le mécanisme de transmission de commande est configuré de sorte que l'actionnement du mécanisme de commande dans la première configuration ou dans la deuxième configuration entraine en rotation l'élément de transmission de commande autour du premier axe de rotation, et permet l'alternance des pieds de stabilisation au sol entre la configuration déployée et la configuration repliée.

Dans un premier mode de réalisation, la première articulation comprend,
- une première tige qui présente un deuxième axe de rotation qui est sensiblement parallèle au premier axe de rotation de l'élément de transmission de commande, la première tige étant couplée à la deuxième portion dudit élément de transmission de commande,
- une première tête pivotante qui présente une section principale qui est montée en rotation sur la première tige et une section de bras qui s'étend depuis la section principale, la première tête pivotante étant reliée au premier pied de stabilisation au sol, et
- un premier embout à rotule qui est monté en rotation sur la première tige et qui est relié, immobilisé en rotation, à la structure de liaison.

Dans un deuxième mode de réalisation, la deuxième articulation comprend,
- une deuxième tige qui présente un troisième axe de rotation qui est sensiblement parallèle au premier axe de rotation de l'élément de transmission de commande, la deuxième tige étant couplée au châssis de plancher porteur,
- une deuxième tête pivotante qui présente une section principale qui est montée en rotation sur la deuxième tige et une section de bras qui s'étend depuis la section principale, la deuxième tête pivotante étant reliée au deuxième pied de stabilisation au sol, et
- un deuxième embout à rotule qui est monté en rotation sur la deuxième tige et qui est relié, immobilisé en rotation, à la structure de liaison.

Dans un premier exemple du premier mode de réalisation et du deuxième mode de réalisation, le premier embout à rotule et le deuxième embout à rotule sont inclinés vers le haut à partir de la structure de liaison jusqu'à, respectivement, la première tige et la deuxième tige.

Dans un deuxième exemple du premier mode de réalisation et du deuxième mode de réalisation, la structure de liaison comprend,
- au moins un premier bras d'extrémité qui est relié au premier embout à rotule,
- au moins un deuxième bras d'extrémité qui est relié au deuxième embout à rotule, et
- un corps central qui est relié entre le premier embout à rotule et le deuxième embout à rotule.

Dans un troisième mode de réalisation, tout ou partie du mécanisme de transmission de commande est configuré pour être monté amovible ou être fixé à demeure sur le châssis de plancher porteur.

Dans un quatrième mode de réalisation, tout ou partie du mécanisme de transmission de commande est configuré pour être monté amovible ou être fixé à demeure sous le châssis de plancher porteur.

Dans un cinquième mode de réalisation, les pieds de stabilisation au sol sont agencés en triangle entre une partie avant et une partie arrière du châssis de plancher porteur.

Dans un sixième mode de réalisation,
- tout ou partie des pieds de stabilisation au sol présentent une forme de L incliné par rapport à un axe longitudinal du châssis de plancher porteur, avec chacun une branche longue et une branche courte, et
- le châssis de plancher porteur présente des découpes, chacune étant agencée pour recevoir la branche courte d'au moins un pied de stabilisation au sol, lorsque le pied de stabilisation au sol est dans la configuration repliée.

L'invention vise aussi une station mobile itinérante robotisée de traitement dentaire ou médical qui comprend,
- au moins un bras robotisé qui est configuré pour déplacer au moins un instrument dentaire ou médical,
- un chariot médical qui comprend un châssis de plancher porteur, le châssis de plancher porteur,
   - ayant un axe longitudinal,
   - présentant une partie avant par rapport à une direction de déplacement du chariot médical et une partie arrière opposée à la partie avant, et
   - étant muni de liaisons au sol conçues pour lui permettre de se déplacer sur le sol, et
- un dispositif de stabilisation selon l'invention.

Dans un premier mode de réalisation, tout ou partie du mécanisme de transmission de commande du dispositif de stabilisation est configuré pour être monté amovible ou être fixé à demeure sous le châssis de plancher porteur.

Dans un deuxième mode de réalisation, tout ou partie du mécanisme de transmission de commande du dispositif de stabilisation est configuré pour être monté amovible ou être fixé à demeure sur le châssis de plancher porteur.

### Brève description des dessins

D'autres caractéristiques et avantages de l'invention seront mieux compris à la lecture de la description qui va suivre et en référence aux dessins annexés, donnés à titre illustratif et nullement limitatif.
[Fig. 1] La figure 1 représente une première vue de perspective, de côté, d'un mode de réalisation d'un chariot médical équipé d'un dispositif de stabilisation selon l'invention, dans la configuration repliée des pieds de stabilisation au sol.
[Fig. 2] La figure 2 représente un mode de réalisation d'un dispositif de stabilisation selon l'invention, dans la configuration déployée des pieds de stabilisation au sol.
[Fig. 3] La figure 3 représente une deuxième vue de perspective, du dessous, du chariot médical de la figure 1.
[Fig. 4] La figure 4 représente un mode de réalisation d'un dispositif de stabilisation selon l'invention, dans la configuration repliée des pieds de stabilisation au sol.
[Fig. 5] La figure 5 représente une troisième vue de perspective, de côté, d'un mode de réalisation d'un chariot médical équipé d'un dispositif de stabilisation selon l'invention, dans la configuration déployée des pieds de stabilisation au sol.
[Fig. 6] La figure 6 représente une quatrième vue de perspective, de côté, d'un mode de réalisation d'un chariot médical équipé d'un dispositif de stabilisation selon l'invention, dans la configuration repliée des pieds de stabilisation au sol.
[Fig. 7] La figure 7 représente un mode de réalisation d'un pied de stabilisation au sol.
[Fig. 8] La figure 8 représente une vue de perspective, du dessous, d'une variante du chariot médical de la figure 1 avec un agencement particulier des pieds de stabilisation au sol de la figure 7, dans la configuration déployée.
[Fig. 9] La figure 9 représente une autre vue de perspective, du dessous, de la variante du chariot médical de la figure 8 avec un agencement particulier des pieds de stabilisation au sol de la figure 7, dans la configuration repliée.
[Fig. 10] La figure 10 représente une vue éclatée d'une première articulation du dispositif de stabilisation selon l'invention.
[Fig. 11] La figure 11 représente une vue éclatée d'une deuxième articulation du dispositif de stabilisation selon l'invention.

Les figures ne respectent pas nécessairement les échelles, notamment en épaisseur, et ce, à des fins d'illustration.

### Description des modes de réalisation

L'un des objectifs de cette invention est de fournir un dispositif de stabilisation pour chariots médicaux.

Pour cela, les inventeurs proposent une structure mécanique à actionnement aisé et qui peut être montée amovible ou être fixée à demeure à un plancher du chariot médical.

Ainsi, l'invention concerne un dispositif de stabilisation pour un chariot médical.

On entend par « chariot », un véhicule ou une remorque qu'un utilisateur, généralement situé derrière ou à côté du chariot, propulse dans une direction de déplacement, et qui permet de transporter des matériaux ou des marchandises sur de courtes distances. Ainsi, un chariot « médical » (également appelé « guéridon de soin » ou « chariot de soin ») est un chariot destiné à recevoir, entre autres, les instruments nécessaires à un acte de soin ou une intervention dentaire ou médicale.

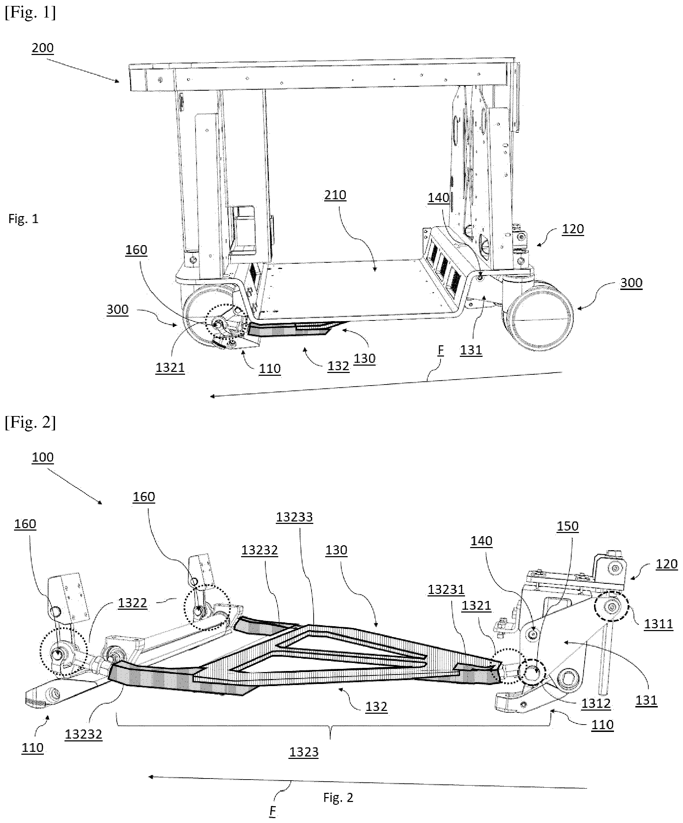

En pratique, dans l'invention, comme illustré sur la figure 1, le chariot médical 200 comprend un châssis de plancher porteur 210.

Le terme « châssis » dans l'expression « châssis de plancher porteur » correspond à un assemblage fixe ou orientable, qui est généralement formé dans un matériau donné (p. ex. du métal et/ou du bois), et qui est destiné à supporter le chariot médical 200 et ce que le chariot médical 200 transporte.

En outre, le terme « plancher » dans l'expression « châssis de plancher porteur » correspond à une plateforme ou une paroi sensiblement horizontale qui délimite la partie inférieure du chariot médical 200.

Enfin, le terme « porteur » dans l'expression « châssis de plancher porteur » correspond au fait que le plancher peut supporter tout ou partie de la charge du chariot médical 200.

De manière classique, comme illustré sur la figure 1, la figure 8 et la figure 9, le châssis de plancher porteur 210 s'étend d'amont en aval dans le sens de la flèche F, le long d'un axe longitudinal, entre une partie arrière, et une partie avant, qui est opposée à la partie arrière.

En outre, le châssis de plancher porteur 210 est muni de liaisons au sol 300 qui sont conçues pour lui permettre de se déplacer sur le sol.

Dans un premier exemple, comme illustré sur la figure 1, la figure 3, la figure 5, la figure 6, la figure 8 et la figure 9, les liaisons au sol 300 sont des roues.

Dans un deuxième exemple, les liaisons au sol 300 sont des chenilles.

Toutefois, on pourra envisager d'utiliser d'autres types de liaisons au sol 300, et ce, sans nécessiter de modifications substantielles de l'invention.

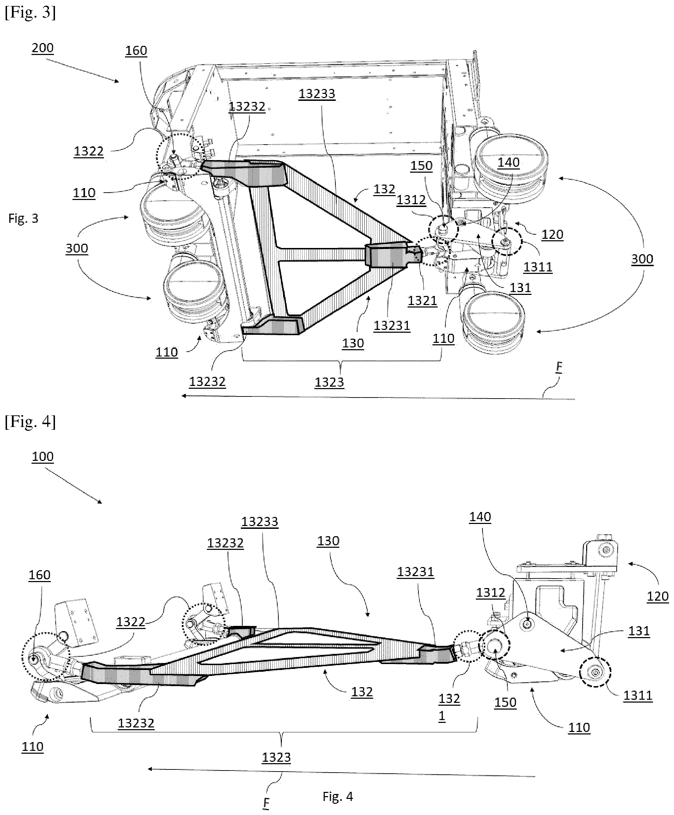

Dans l'invention, comme illustré sur la figure 2 et la figure 3, le dispositif de stabilisation 100 comprend des pieds de stabilisation au sol 110, un mécanisme de commande 120 et un mécanisme de transmission de commande 130.

Le terme « pied » dans l'expression « pieds de stabilisation au sol », correspond à une pièce formée dans un matériau donné (p. ex. du métal et/ou du bois) et qui sert de support du chariot médical 200 par rapport au sol.

Dans l'invention, les pieds de stabilisation au sol 110 sont configurés pour supporter le châssis de plancher porteur 210.

Dans une première mise en oeuvre particulière des pieds de stabilisation au sol 110, comme illustré sur la figure 3, ceux-ci sont agencés en triangle entre une partie avant et une partie arrière du châssis de plancher porteur 210.

Dans un premier exemple de la première mise en oeuvre particulière des pieds de stabilisation au sol 110, on dispose au moins un pied avant de stabilisation au sol 110 au niveau de la partie avant du châssis de plancher porteur 210 et au moins deux pieds arrière de stabilisation au sol 110 au niveau de la partie arrière du châssis de plancher porteur 210.

Dit autrement, le premier exemple de la première mise en oeuvre particulière des pieds de stabilisation au sol 110 couvre la disposition d'un, deux ou plus de deux pieds avant de stabilisation au sol 110 au niveau de la partie avant du châssis de plancher porteur 210, et deux ou plus de deux pieds arrière de stabilisation au sol 110 au niveau de la partie arrière du châssis de plancher porteur 210.

Dans un mode de réalisation du premier exemple de la première mise en oeuvre particulière des pieds de stabilisation au sol 110, on dispose au moins deux liaisons au sol 300 avant, de part et d'autre du pied avant de stabilisation, et au moins une liaison au sol 300 arrière entre les pieds arrière de stabilisation au sol 110.

Dans un deuxième exemple de la première mise en oeuvre particulière des pieds de stabilisation au sol 110, comme illustré sur la figure 3, on dispose au moins deux pieds avant de stabilisation au sol 110 au niveau de la partie avant du châssis de plancher porteur 210 et au moins un pied arrière de stabilisation au sol 110 au niveau de la partie arrière du châssis de plancher porteur 210.

Dit autrement, le deuxième exemple de la première mise en oeuvre particulière des pieds de stabilisation au sol 110 couvre la disposition de deux ou plus de deux pieds avant de stabilisation au sol 110 au niveau de la partie avant du châssis de plancher porteur 210, et un, deux ou plus de deux pieds arrière de stabilisation au sol 110 au niveau de la partie arrière du châssis de plancher porteur 210.

Dans un mode de réalisation du deuxième exemple de la première mise en oeuvre particulière des pieds de stabilisation au sol 110, on dispose au moins une liaison au sol 300 avant entre les pieds avant de stabilisation et au moins deux liaisons au sol 300 arrière, de part et d'autre du pied arrière de stabilisation au sol 110.

Toutefois, on pourra envisager d'autres agencements de pieds de stabilisation en combinaison avec différents agencements de liaisons au sol 300, et ce, sans nécessiter de modifications substantielles de l'invention.

Dans une deuxième mise en oeuvre particulière des pieds de stabilisation au sol 110, ceux-ci sont agencés de manière symétrique autour d'un axe médian longitudinal du châssis de plancher porteur 210.

Dans une troisième mise en oeuvre particulière des pieds de stabilisation au sol 110, ceux-ci sont agencés de manière symétrique autour d'un axe qui est perpendiculaire à l'axe médian longitudinal du châssis de plancher porteur 210.

Dans une quatrième mise en oeuvre particulière des pieds de stabilisation au sol 110, au moins deux pieds de stabilisation au sol 110 sont formés d'un seul tenant.

Dit autrement, la quatrième mise en oeuvre particulière des pieds de stabilisation au sol 110 couvre le fait que deux ou plus de deux pieds de stabilisation au sol 110 sont formés d'un seul tenant.

Dans l'invention, les pieds de stabilisation au sol 110 sont convertibles entre une configuration déployée et une configuration repliée.

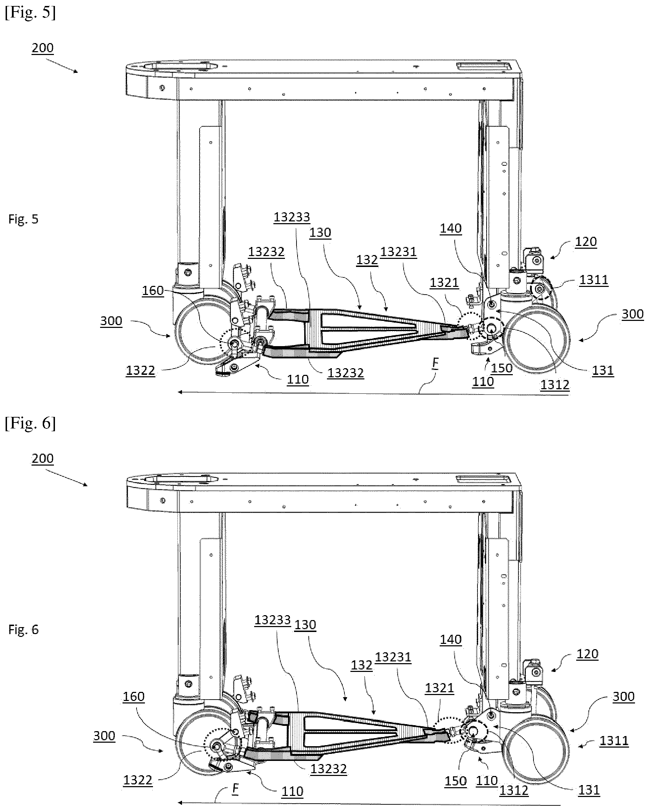

Dans la configuration déployée, comme illustré sur la figure 2 et la figure 5, les pieds de stabilisation au sol 110 sont en appui sur le sol, de façon stable, de manière à supporter, ensemble, tout ou partie de ce que le châssis de plancher porteur 210 supporte.

Dans une mise en oeuvre particulière de la configuration déployée, les pieds de stabilisation au sol 110 soulèvent le châssis de plancher porteur 210 par rapport au sol de sorte que les liaisons au sol 300 ne sont pas en contact avec le sol et de manière à soulager les liaisons au sol 300.

Dans un exemple de cette mise en oeuvre particulière de la configuration déployée, les pieds de stabilisation au sol 110 soulèvent le châssis de plancher porteur 210 dans une plage comprise entre 1 mm et 20 mm par rapport au sol, de préférence entre 2 mm et 5 mm par rapport au sol.

Toutefois, on pourra envisager que dans la configuration déployée, les liaisons au sol 300 effleurent le sol, et ce, sans nécessiter de modifications substantielles de l'invention.

Dans la configuration repliée, comme illustré sur la figure 1, la figure 3, la figure 4 et la figure 6, les pieds de stabilisation au sol 110 sont décollés du sol de manière à assurer un escamotage au moins partiel.

On entend par « escamotage », le fait de faire disparaître, de replier ou de ranger les pieds de stabilisation au sol 110.

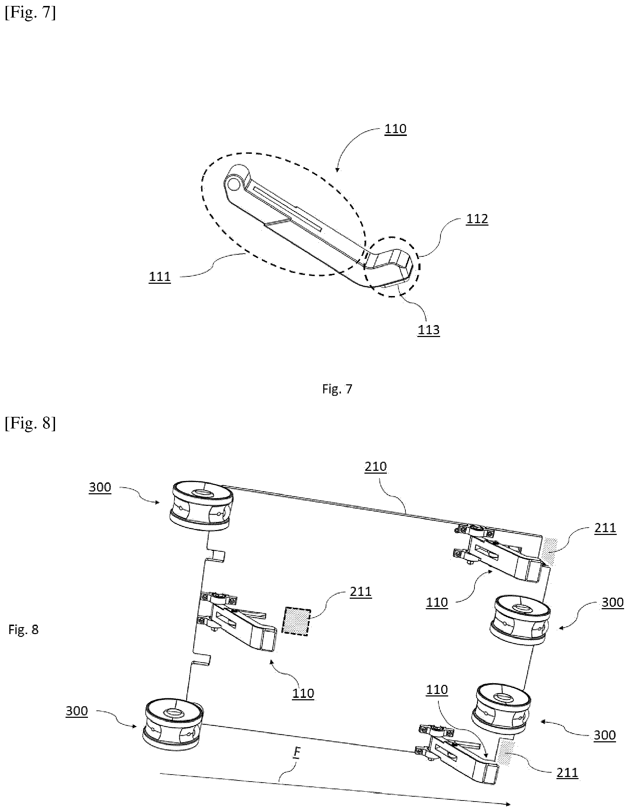

Dans une mise en oeuvre particulière des pieds de stabilisation au sol 110, comme illustré sur la figure 7, tout ou partie de ceux-ci présentent une forme de L incliné par rapport à l'axe longitudinal du châssis de plancher porteur 210.

De manière classique, chaque pied de stabilisation au sol 110 en forme de L incliné présente une branche longue 111 et une branche courte 112.

Par ailleurs, dans un exemple comme illustré sur la figure 7, chaque pied de stabilisation au sol 110 en forme de L incliné comprend un patin 113.

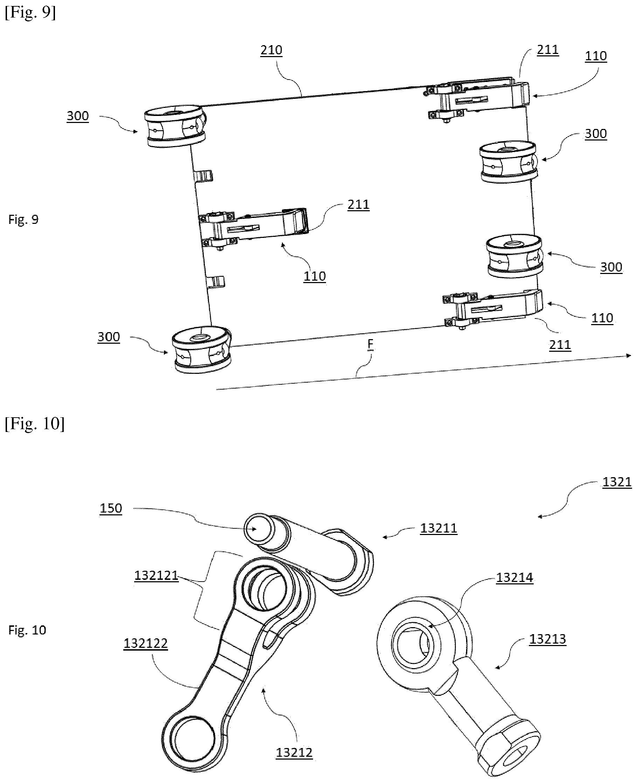

En outre, comme illustré sur la figure 8 et sur la figure 9, le châssis de plancher porteur 210 présente des découpes 211.

En pratique, chaque découpe 211 est agencée pour recevoir la branche courte 112 d'au moins un pied de stabilisation au sol 110, lorsque le pied de stabilisation au sol 110 est dans la configuration repliée.

Dans l'invention, le mécanisme de commande 120 est déplaçable entre une première position et une deuxième position, en réponse à au moins un mouvement de translation ou un mouvement de rotation.

Dit autrement, le mécanisme de commande 120 est déplaçable entre une première position et une deuxième position, en réponse à soit un mouvement de translation, soit un mouvement de rotation, soit une combinaison entre un ou plusieurs mouvements de translation et un ou plusieurs mouvements de rotation.

Dans un premier exemple d'une mise en oeuvre du mécanisme de commande 120, celui-ci est mis en translation par l'actionnement d'un organe de direction tel un curseur translatif.

Toutefois, on pourra envisager d'utiliser d'autres mécanismes d'actionnement de translation du mécanisme de commande 120, qu'ils soient à actionnement manuel ou automatique, et ce, sans nécessiter de modifications substantielles de l'invention.

Dans un deuxième exemple d'une mise en oeuvre du mécanisme de commande 120, celui-ci est mis en rotation par l'actionnement d'un organe de direction sélectionné parmi un volant rotatif, un levier pivotant, un guidon pivotant, une pédale pivotante, une manivelle, et un gouvernail.

Toutefois, on pourra envisager d'utiliser d'autres mécanismes d'actionnement de rotation du mécanisme de commande 120, et ce, sans nécessiter de modifications substantielles de l'invention.

Toujours dans l'invention, comme illustré sur la figure 1, la figure 2, la figure 3, la figure 4, la figure 5 et la figure 6, le mécanisme de transmission de commande 130 comprend un ensemble de liaison 132 et au moins un élément de transmission de commande 131.

Dit autrement, le mécanisme de transmission de commande 130 comprend un ensemble de liaison 132 et un, deux ou plus de deux éléments de transmission de commande 131.

Dans une mise en oeuvre particulière de l'élément de transmission de commande 131, comme illustré sur la figure 2, la figure 3, la figure 4, la figure 5 et la figure 6, celui-ci présente une première portion 1311 dudit élément de transmission de commande 131 et une deuxième portion 1312 dudit élément de transmission de commande 131.

En pratique, la première portion 1311 dudit élément de transmission de commande 131 est reliée cinématiquement, de manière directe ou indirecte, au mécanisme de commande 120.

On entend par « cinématiquement », un mode de liaison mécanique qui permet la description d'un mouvement cinématique, c'est-à-dire un angle de rotation, une amplitude de rotation, un vecteur de déplacement, une amplitude de translation, une vitesse ou une accélération.

Puis, la deuxième portion 1312 dudit élément de transmission de commande 131 est reliée cinématiquement, de manière articulée, aux pieds de stabilisation au sol 110 via l'ensemble de liaison 132.

Par ailleurs, l'élément de transmission de commande 131 est monté rotatif autour d'un premier axe de rotation 140 de manière à être entraîné en rotation autour du premier axe de rotation 140, dans un premier sens de rotation et dans un deuxième sens de rotation opposé au premier sens de rotation.

Dans un mode de réalisation de l'élément de transmission de commande 131, celui-ci se présente sous la forme d'au moins une plaque.

On entend par « plaque », un morceau d'un matériau rigide, globalement plat, dont l'épaisseur est faible, comparée aux deux autres dimensions, et ce, que la section droite soit rectiligne ou non, par exemple en créneaux ou sinusoïdal tel qu'une tôle ondulée.

Dans un exemple, comme illustré sur la figure 2, la figure 3, la figure 4, la figure 5 et la figure 6, l'élément de transmission de commande 131 présente une forme triangulaire.

Dans cet exemple, la première portion 1311 dudit élément de transmission de commande 131, la deuxième portion 1312 dudit élément de transmission de commande 131 et le premier axe de rotation 140 sont respectivement disposés à un sommet de l'élément de transmission de commande 131 en forme triangulaire.

Toutefois, on pourra envisager d'utiliser d'autres formes géométriques (p. ex. circulaire, semi-circulaire, carrée, rectangulaire, tronconique, conique, polygonale, hexagonale, trapézoïdale, elliptique, semi-elliptique, convexe, en croix et en étoile), et ce, sans nécessiter de modifications substantielles de l'invention.

Dans l'invention, le mécanisme de transmission de commande 130 est configuré de sorte que l'actionnement du mécanisme de commande 120 dans la première configuration ou dans la deuxième configuration entraine en rotation l'élément de transmission de commande 131 autour du premier axe de rotation 140, et permet l'alternance des pieds de stabilisation au sol 110 entre la configuration déployée et la configuration repliée.

On entend par « alternance », une succession de l'état des pieds de stabilisation au sol 110, dans un ordre régulier, dans le temps ou l'espace.

La figure 2 et la figure 5 illustrent la configuration déployée des pieds de stabilisation au sol 110.

Dans cette configuration déployée des pieds de stabilisation au sol 110, on a actionné le mécanisme de commande 120 dans la première position de sorte que l'élément de transmission de commande 131 est entraîné dans le premier sens de rotation autour du premier axe de rotation 140.

En pratique, dans l'exemple de la figure 2 et de la figure 5, la première portion 1311 dudit élément de transmission de commande 131 se trouve en position haute par rapport au sol tandis que la deuxième portion 1312 dudit élément de transmission de commande 131 se trouve en position basse par rapport au sol, de sorte que les pieds de stabilisation au sol 110 sont en appui sur le sol, de façon stable.

Toutefois, on pourra envisager que dans la configuration déployée des pieds de stabilisation au sol 110, la première portion 1311 dudit élément de transmission de commande 131 se trouve en position basse par rapport au sol tandis que la deuxième portion 1312 dudit élément de transmission de commande 131 se trouve en position haute par rapport au sol, et ce, sans nécessiter de modifications substantielles de l'invention, de sorte que les pieds de stabilisation au sol 110 sont en appui sur le sol, de façon stable.

La figure 4 et la figure 6 illustrent la configuration repliée des pieds de stabilisation au sol 110.

Dans cette configuration repliée des pieds de stabilisation au sol 110, on a actionné le mécanisme de commande 120 dans la deuxième position de sorte que l'élément de transmission de commande 131 est entraîné dans le deuxième sens de rotation autour du premier axe de rotation 140.

En pratique, dans l'exemple de la figure 4 et de la figure 6, la première portion 1311 dudit élément de transmission de commande 131 se trouve en position basse par rapport au sol tandis que la deuxième portion 1312 dudit élément de transmission de commande 131 se trouve en position haute par rapport au sol, de sorte que les pieds de stabilisation au sol 110 sont décollés du sol.

Toutefois, on pourra envisager que dans la configuration repliée des pieds de stabilisation au sol 110, la première portion 1311 dudit élément de transmission de commande 131 se trouve en position haute par rapport au sol tandis que la deuxième portion 1312 dudit élément de transmission de commande 131 se trouve en position basse par rapport au sol, et ce, sans nécessiter de modifications substantielles de l'invention, de sorte que les pieds de stabilisation au sol 110 sont décollés du sol.

Toujours dans l'invention, dans une mise en oeuvre particulière de l'ensemble de liaison 132, comme illustré sur la figure 1, la figure 2, la figure 3, la figure 4, la figure 5 et la figure 6, celui-ci comprend au moins deux articulations, à savoir au moins une première articulation 1321, à au moins un seul degré de liberté, et au moins une deuxième articulation 1322, à au moins un seul degré de liberté, et une structure de liaison 1323.

Dit autrement, dans cette mise en oeuvre particulière de l'ensemble de liaison 132, celui-ci comprend une structure de liaison 1323, un, deux ou plus de deux premières articulations 1321, à un, deux, ou plus de deux degrés de liberté, et un, deux, ou plus de deux deuxièmes articulations 1322, à un, deux, ou plus de deux degrés de liberté.

On entend par « articulation », un élément de liaison pour pièces mécaniques qui permet un ou plusieurs mouvements relatifs indépendants des pièces mécaniques les unes par rapport aux autres.

On entend par « degré de liberté », le fait que la première articulation 1321 et la deuxième articulation 1322 autorisent un, deux ou plus de deux mouvements relatifs indépendants des pièces mécaniques qu'elles lient.

Dans un premier exemple, la première articulation 1321 et/ou la deuxième articulation 1322 sont des liaisons glissières.

Dans un deuxième exemple, la première articulation 1321 et/ou la deuxième articulation 1322 sont des liaisons glissières hélicoïdales.

Dans un troisième exemple, la première articulation 1321 et/ou la deuxième articulation 1322 sont des liaisons pivots.

Dans un quatrième exemple, la première articulation 1321 et/ou la deuxième articulation 1322 sont des liaisons pivots glissants.

Dans un cinquième exemple, la première articulation 1321 et/ou la deuxième articulation 1322 sont des liaisons rotules.

Dans un sixième exemple, la première articulation 1321 et/ou la deuxième articulation 1322 sont des liaisons rotules à doigt.

Dans la mise en oeuvre particulière de l'ensemble de liaison 132, la première articulation 1321 est directement reliée à la deuxième portion 1312 dudit élément de transmission de commande 131 et à au moins un premier pied de stabilisation au sol 110.

Dit autrement, dans cette mise en oeuvre particulière de l'ensemble de liaison 132, la première articulation 1321 est directement reliée à la deuxième portion 1312 dudit élément de transmission de commande 131 et à un, deux ou plus de deux premiers pieds de stabilisation au sol 110.

Dans un premier mode de réalisation de la mise en oeuvre particulière de l'ensemble de liaison 132, comme illustré sur la figure 10, la première articulation 1321, comprend une première tige 13211, une première tête pivotante 13212 et un premier embout à rotule 13213 monté autour d'une rotule 13214.

On entend par « tige », une pièce, de section constante ou variable, qui présente une élongation dans une direction et qui peut être inscrite dans un cylindre ou un prisme d'une longueur désirée et d'un diamètre désiré.

On entend par « embout à rotule » (également connu sous le nom de « palier à embout »), un composant mécanique formé d'un embout et d'une rotule et qui permet d'établir une connexion entre au moins deux éléments fixes ou mobiles. Un embout à rotule sert généralement à fournir un mouvement de rotation et d'oscillation sans aucun jeu. Un embout à rotule sert également à transmettre la traction et la poussée de liaisons et de bras de levier. On utilise généralement les embouts à rotule sur les extrémités des pistons hydrauliques ou pneumatiques ou à la base des vérins hydrauliques.

Dans le premier mode de réalisation de la mise en oeuvre particulière de l'ensemble de liaison 132, comme illustré sur la figure 10, la première tige 13211 présente un deuxième axe de rotation 150 qui est sensiblement parallèle au premier axe de rotation 140 de l'élément de transmission de commande 131.

En outre, la première tige 13211 est couplée à la deuxième portion 1312 dudit élément de transmission de commande 131.

Toujours dans le premier mode de réalisation de la mise en oeuvre particulière de l'ensemble de liaison 132, la première tête pivotante 13212 est en forme de Y et présente une section principale 132121 qui est montée en rotation sur la première tige 13211, ainsi qu'une première section de bras 132122 qui s'étend depuis la section principale 132121.

En outre, la première tête pivotante 13212 est reliée au premier pied de stabilisation au sol 110 via la première section de bras 132122.

Dans un exemple, la première section de bras 132122 de la première tête pivotante 13212 est reliée pivotante au premier pied de stabilisation au sol 110.

Toutefois, on pourra envisager d'utiliser d'autres liaisons, et ce, sans nécessiter de modifications substantielles de l'invention.

Enfin, dans le premier mode de réalisation de la mise en oeuvre particulière de l'ensemble de liaison 132, le premier embout à rotule 13213 est, d'une part, monté en rotation sur la première tige 13211. D'autre part, le premier embout à rotule 13213 est relié, immobilisé en rotation, à la structure de liaison 1323.

Dans un aspect du premier mode de réalisation de la mise en oeuvre particulière de l'ensemble de liaison 132, le premier embout à rotule 13213 est incliné vers le haut à partir de la structure de liaison 1323 jusqu'à la première tige 13211.

Dans un exemple de l'aspect du premier mode de réalisation de la mise en oeuvre particulière de l'ensemble de liaison 132, le premier embout à rotule 13213 est incliné selon un angle d'inclinaison prédéterminé qui est compris entre 10° et 20°.

Toutefois, selon les besoins, on pourra envisager d'autres valeurs, et ce, sans nécessiter de modifications substantielles de l'invention.

Toujours dans la mise en oeuvre particulière de l'ensemble de liaison 132, la deuxième articulation 1322 est directement reliée à au moins un deuxième pied de stabilisation au sol 110.

Dit autrement, dans cette mise en oeuvre particulière de l'ensemble de liaison 132, la deuxième articulation 1322 est directement reliée à un, deux ou plus de deux deuxièmes pieds de stabilisation au sol 110.

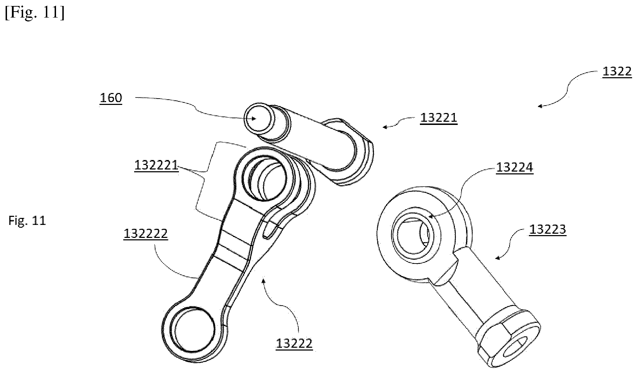

Dans un deuxième mode de réalisation de la mise en oeuvre particulière de l'ensemble de liaison 132, comme illustré sur la figure 11, la deuxième articulation 1322 comprend une deuxième tige 13221, une deuxième tête pivotante 13222 et un deuxième embout à rotule 13223 monté autour d'une rotule 13224.

Dans le deuxième mode de réalisation de la mise en oeuvre particulière de l'ensemble de liaison 132, la deuxième tige 13221 présente un troisième axe de rotation 160 qui est sensiblement parallèle au premier axe de rotation 140 de l'élément de transmission de commande 131.

En outre, la deuxième tige 13221 est couplée au châssis de plancher porteur 210.

Toujours dans le deuxième mode de réalisation de la mise en oeuvre particulière de l'ensemble de liaison 132, la deuxième tête pivotante 13222 est en forme de Y et présente une section principale 132221 qui est montée en rotation sur la deuxième tige, ainsi qu'une deuxième section de bras 132222 qui s'étend depuis la section principale 132221.

En outre, la deuxième tête pivotante 13222 est reliée au deuxième pied de stabilisation au sol 110 via la deuxième section de bras 132222.

Dans un exemple, la deuxième section de bras 132222 de la deuxième tête pivotante 13222 est reliée pivotante au deuxième pied de stabilisation au sol 110.

Toutefois, on pourra envisager d'utiliser d'autres liaisons, et ce, sans nécessiter de modifications substantielles de l'invention.

Enfin, dans le deuxième mode de réalisation de la mise en oeuvre particulière de l'ensemble de liaison 132, le deuxième embout à rotule 13223 est, d'une part, monté en rotation sur la deuxième tige 13221. D'autre part, le deuxième embout à rotule 13223 est relié, immobilisé en rotation, à la structure de liaison 1323.

Dans un aspect du deuxième mode de réalisation de la mise en oeuvre particulière de l'ensemble de liaison 132, le deuxième embout à rotule 13223 est incliné vers le haut à partir de la structure de liaison 1323 jusqu'à la deuxième tige 13221.

Dans un exemple de l'aspect du deuxième mode de réalisation de la mise en oeuvre particulière de l'ensemble de liaison 132, le deuxième embout à rotule 13223 est incliné selon un angle d'inclinaison prédéterminé qui est compris entre 10° et 20°.

Toutefois, selon les besoins, on pourra envisager d'autres valeurs, et ce, sans nécessiter de modifications substantielles de l'invention.

Finalement, dans la mise en oeuvre particulière de l'ensemble de liaison 132, la structure de liaison 1323 est directement reliée entre la première articulation 1321 et la deuxième articulation 1322.

Dans un mode de réalisation de la mise en oeuvre particulière de l'ensemble de liaison 132, la structure de liaison 1323 comprend au moins un premier bras d'extrémité 13231, au moins un deuxième bras d'extrémité 13232 et un corps central 13233.

Dit autrement, dans ce mode de réalisation de la mise en oeuvre particulière de l'ensemble de liaison 132, la structure de liaison 1323 comprend un corps central 13233, un, deux ou plus de deux premiers bras d'extrémité 13231, et un, deux ou plus de deux deuxièmes bras d'extrémité 13232.

En pratique, le premier bras d'extrémité 13231 est relié au premier embout à rotule 13213, le deuxième bras d'extrémité 13232 est relié au deuxième embout à rotule 13223, et le corps central 13233 est relié entre le premier embout à rotule 13213 et le deuxième embout à rotule 13223.

Dans un exemple du corps central 13233, comme illustré sur la figure 2, la figure 3, la figure 4, la figure 5 et la figure 6, celui-ci présente une forme triangulaire.

Toutefois, on pourra envisager d'utiliser d'autres formes géométriques (p. ex. carrée, rectangulaire, polygonale, hexagonale, trapézoïdale, en croix et en étoile), et ce, sans nécessiter de modifications substantielles de l'invention.

L'invention concerne également une station mobile itinérante robotisée de traitement dentaire ou médical.

En particulier, la station mobile itinérante robotisée de traitement dentaire ou médical comprend au moins un bras robotisé, un chariot médical 200 et un dispositif de stabilisation 100, selon l'invention.

Dit autrement, la station mobile itinérante robotisée de traitement dentaire ou médical comprend un chariot médical 200 et un dispositif de stabilisation 100, selon l'invention et un, deux ou plus de deux bras robotisés.

En pratique, le bras robotisé est configuré pour déplacer au moins un instrument dentaire ou médical.

Dit autrement, le bras robotisé est configuré pour déplacer un, deux ou plus de deux instruments dentaires ou médicaux.

Enfin, le chariot médical 200 comprend un châssis de plancher porteur 210 qui a un axe longitudinal F, et qui présente une partie avant par rapport à une direction de déplacement du chariot médical 200 et une partie arrière opposée à la partie avant, et étant muni de liaisons au sol 300 conçues pour se déplacer sur le sol.

Avec une telle station mobile itinérante robotisée de traitement dentaire ou médical, lorsque les pieds de stabilisation au sol 110 sont en configuration déployée, la station mobile itinérante robotisée de traitement dentaire ou médical est immobilisée de manière stable de sorte que le bras robotisé est dans une disposition de travail pour déplacer l'instrument dentaire ou médical.

En outre, lorsque les pieds de stabilisation au sol 110 sont en configuration repliée, la station mobile itinérante robotisée de traitement dentaire ou médical est mobile sur le sol de sorte que le bras robotisé est dans une disposition de repos, sans déplacement de l'instrument dentaire ou médical.

Dans une première mise en oeuvre particulière de la station mobile itinérante robotisée de traitement dentaire ou médical, tout ou partie du mécanisme de transmission de commande 130 est configuré pour être monté amovible ou être fixé à demeure sur le châssis de plancher porteur 210.

Dans une deuxième mise en oeuvre particulière de la station mobile itinérante robotisée de traitement dentaire ou médical, comme illustré sur la figure 1, la figure 3, la figure 5 et la figure 6, tout ou partie du mécanisme de transmission de commande 130 est configuré pour être monté amovible ou être fixé à demeure sous le châssis de plancher porteur 210.

Nous avons décrit et illustré l'invention. Toutefois, l'invention ne se limite pas aux formes de réalisations que nous avons présentées. En effet, de nombreuses combinaisons des variantes, alternatives, modes de réalisation et mises en oeuvre, peuvent être envisagées sans nécessiter de modifications substantielles de l'invention. Ainsi, un expert du domaine peut déduire d'autres variantes, alternatives, modes de réalisation et mises en oeuvre, à la lecture de la description et des figures annexées et en fonction des contraintes économiques, ergonomiques, dimensionnelles à respecter.

L'invention peut faire l'objet de nombreuses variantes et applications autres que celles décrites ci-dessus. En particulier, sauf indication contraire, les différentes caractéristiques structurelles et fonctionnelles de chaque mise en oeuvre particulière décrite ci-dessus ne doivent pas être considérées comme combinées et/ou étroitement et/ou inextricablement liées les unes aux autres, mais, au contraire, comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

## Revendications

1. Dispositif de stabilisation (100) pour un chariot médical (200) comprenant un châssis de plancher porteur (210), le dispositif de stabilisation (100) comprenant,
- des pieds de stabilisation au sol (110) qui sont configurés pour supporter le châssis de plancher porteur (210) et qui sont convertibles entre une configuration déployée dans laquelle les pieds de stabilisation au sol (110) sont en appui sur le sol, de façon stable, de manière à supporter, ensemble, tout ou partie de ce que le châssis de plancher porteur (210) supporte, et une configuration repliée dans laquelle les pieds de stabilisation au sol (110) sont décollés du sol de manière à assurer un escamotage au moins partiel,
- un mécanisme de commande (120) qui est déplaçable entre une première position et une deuxième position, en réponse à au moins un mouvement de translation ou un mouvement de rotation, et
- un mécanisme de transmission de commande (130) qui comprend,
- un ensemble de liaison (132), et
- au moins un élément de transmission de commande (131) qui présente une première portion (1311) dudit élément de transmission de commande (131) reliée cinématiquement, de manière directe ou indirecte, au mécanisme de commande, et une deuxième portion (1312) dudit élément de transmission de commande (131) reliée cinématiquement, de manière articulée, aux pieds de stabilisation au sol (110) via l'ensemble de liaison (132), l'élément de transmission de commande (131) étant monté rotatif autour d'un premier axe de rotation (140) de manière à être entraîné en rotation dans un premier sens de rotation et dans un deuxième sens de rotation opposé au premier sens de rotation,
ledit ensemble de liaison (132) comprenant,
- au moins une première articulation (1321), à au moins un degré de liberté, qui est directement reliée à la deuxième portion (1312) dudit élément de transmission de commande (131) et à au moins un premier pied de stabilisation au sol (110),
- au moins une deuxième articulation (1322), à au moins un degré de liberté, qui est directement reliée à au moins un deuxième pied de stabilisation au sol (110), et
- une structure de liaison (1323) qui est reliée entre la première articulation (1321) et la deuxième articulation (1322),
dans lequel,
le mécanisme de transmission de commande (130) est configuré de sorte que l'actionnement du mécanisme de commande (120) dans la première configuration ou dans la deuxième configuration entraine en rotation l'élément de transmission de commande (131) autour du premier axe de rotation, et permet l'alternance des pieds de stabilisation au sol (110) entre la configuration déployée et la configuration repliée.

2. Dispositif de stabilisation (100) selon la revendication 1, dans lequel la première articulation (1321) comprend,
- une première tige (13211) qui présente un deuxième axe de rotation (150) qui est sensiblement parallèle au premier axe de rotation (140) de l'élément de transmission de commande (131), la première tige (13211) étant couplée à la deuxième portion (1312) dudit élément de transmission de commande (131),
- une première tête pivotante (13212) qui présente une section principale (132121) qui est montée en rotation sur la première tige (13211) et une première section de bras (132122) qui s'étend depuis la section principale (132121), la première tête pivotante (13212) étant reliée au premier pied de stabilisation au sol (110), et
- un premier embout à rotule (13213) qui est monté en rotation sur la première tige (13211) et qui est relié, immobilisé en rotation, à la structure de liaison 1323.

3. Dispositif de stabilisation (100) selon l'une quelconque des revendications 1 à 2, dans lequel la deuxième articulation (1322) comprend,
- une deuxième tige (13221) qui présente un troisième axe de rotation (160) qui est sensiblement parallèle au premier axe de rotation (140) de l'élément de transmission de commande (131), la deuxième tige (13221) étant couplée au châssis de plancher porteur (210),
- une deuxième tête pivotante (13222) qui présente une section principale (132221) qui est montée en rotation sur la deuxième tige (13221) et une deuxième section de bras (132222) qui s'étend depuis la section principale (132221), la deuxième tête pivotante (13222) étant reliée au deuxième pied de stabilisation au sol (110), et
- un deuxième embout à rotule (13223) qui est monté en rotation sur la deuxième tige et qui est relié, immobilisé en rotation, à la structure de liaison (1323).

4. Dispositif de stabilisation (100) selon la revendication 3, dans lequel le premier embout à rotule (13213) et le deuxième embout à rotule (13223) sont inclinés vers le haut à partir de la structure de liaison (1323) jusqu'à, respectivement, la première tige (13211) et la deuxième tige (13221).

5. Dispositif de stabilisation (100) selon l'une quelconque des revendications 1 à 4, dans lequel la structure de liaison (1323) comprend,
- au moins un premier bras d'extrémité (13231) qui est relié au premier embout à rotule (13213),
- au moins un deuxième bras d'extrémité (13232) qui est relié au deuxième embout à rotule (13223), et
- un corps central (13233) qui est relié entre le premier embout à rotule (13213) et le deuxième embout à rotule (13223).

6. Dispositif de stabilisation (100) selon l'une quelconque des revendications 1 à 5, dans lequel tout ou partie du mécanisme de transmission de commande (130) est configuré pour être monté amovible ou être fixé à demeure sur le châssis de plancher porteur (210).

7. Dispositif de stabilisation (100) selon l'une quelconque des revendications 1 à 6, dans lequel tout ou partie du mécanisme de transmission de commande (130) est configuré pour être monté amovible ou être fixé à demeure sous le châssis de plancher porteur (210).

8. Dispositif de stabilisation (100) selon l'une quelconque des revendications 1 à 7, dans lequel les pieds de stabilisation au sol (110) sont agencés en triangle entre une partie avant et une partie arrière du châssis de plancher porteur (210).

9. Dispositif de stabilisation (100) selon l'une quelconque des revendications 1 à 8, dans lequel,
- tout ou partie des pieds de stabilisation au sol (110) présentent une forme de L incliné par rapport à un axe longitudinal du châssis de plancher porteur (210), avec chacun une branche longue (111) et une branche courte (112), et
- le châssis de plancher porteur (210) présente des découpes (211), chacune étant agencée pour recevoir la branche courte (112) d'au moins un pied de stabilisation au sol (110), lorsque le pied de stabilisation au sol (110) est dans la configuration repliée.

10. Station mobile itinérante robotisée de traitement dentaire ou médical comprenant,
- au moins un bras robotisé qui est configuré pour déplacer au moins un instrument dentaire ou médical,
- un chariot médical (200) qui comprend un châssis de plancher porteur (210), le châssis de plancher porteur (210),
- ayant un axe longitudinal, F,
- présentant une partie avant par rapport à une direction de déplacement du chariot médical (200) et une partie arrière opposée à la partie avant, et
- étant muni de liaisons au sol (300) conçues pour lui permettre de se déplacer sur le sol, et
- un dispositif de stabilisation (100) selon l'une quelconque des revendications 1 à 9.

11. Station mobile itinérante robotisée de traitement dentaire ou médical selon la revendication 10, dans lequel tout ou partie du mécanisme de transmission de commande (130) du dispositif de stabilisation (100) est configuré pour être monté amovible ou être fixé à demeure sous le châssis de plancher porteur (210).

12. Station mobile itinérante robotisée de traitement dentaire ou médical selon la revendication 10, dans lequel tout ou partie du mécanisme de transmission de commande (130) du dispositif de stabilisation (100) est configuré pour être monté amovible ou être fixé à demeure sur le châssis de plancher porteur (210).

## Patentansprüche

1. Stabilisierungsvorrichtung (100) für einen medizinischen Wagen (200), umfassend einen tragenden Bodenrahmen (210), wobei die Stabilisierungsvorrichtung (100) umfasst
- Bodenstabilisierungsfüße (110), die ausgelegt sind, um den tragenden Bodenrahmen (210) abzustützen und die zwischen einer ausgefahrenen Konfiguration, in der die Bodenstabilisierungsfüße (110) stabil auf dem Boden aufliegen, so dass sie zusammen alles oder einen Teil dessen abstützen, was der tragende Bodenrahmen (210) abstützt, und einer eingeklappten Konfiguration, in der die Bodenstabilisierungsfüße (110) vom Boden abgehoben sind, so dass ein zumindest teilweises Einziehen gewährleistet ist, konvertierbar sind,
- einen Steuermechanismus (120), der als Reaktion auf mindestens eine Translationsbewegung oder eine Drehbewegung zwischen einer ersten Position und einer zweiten Position beweglich ist, und
- einen Steuerungsübertragungsmechanismus (130), der umfasst
- eine Verbindungsanordnung (132), und
- mindestens ein Steuerungsübertragungselement (131), das einen ersten Abschnitt (1311) des Steuerungsübertragungselements (131) aufweist, der kinematisch direkt oder indirekt mit dem Steuermechanismus verbunden ist, und einen zweiten Abschnitt (1312) des Steuerungsübertragungselements (131), der kinematisch gelenkig mit den Bodenstabilisierungsfüßen (110) über die Verbindungsanordnung (132) verbunden ist, wobei das Steuerungsübertragungselement (131) um eine erste Drehachse (140) drehbar angebracht ist, so dass es in einer ersten Drehrichtung und in einer zweiten Drehrichtung, die der ersten Drehrichtung entgegengesetzt ist, rotatorisch angetrieben wird, wobei die Verbindungsanordnung (132) umfasst
- mindestens ein erstes Gelenk (1321) mit mindestens einem Freiheitsgrad, das direkt mit dem zweiten Abschnitt (1312) des Steuerungsübertragungselements (131) und mit mindestens einem ersten Bodenstabilisierungsfuß (110) verbunden ist,
- mindestens ein zweites Gelenk (1322) mit mindestens einem Freiheitsgrad, das direkt mit mindestens einem zweiten Bodenstabilisierungsfuß (110) verbunden ist, und
- eine Verbindungsstruktur (1323), die zwischen dem ersten Gelenk (1321) und dem zweiten Gelenk (1322) verbunden ist,
wobei
der Steuerübertragungsmechanismus (130) derart ausgelegt ist, dass die Betätigung des Steuermechanismus (120) in der ersten Konfiguration oder in der zweiten Konfiguration das Steuerungsübertragungselement (131) um die erste Drehachse dreht, und den Wechsel der Bodenstabilisierungsfüße (110) zwischen der ausgefahrenen und der eingeklappten Konfiguration ermöglicht.

2. Stabilisierungsvorrichtung (100) nach Anspruch 1, wobei das erste Gelenk (1321) umfasst
- eine erste Stange (13211), die eine zweite Drehachse (150) aufweist, die im Wesentlichen parallel zur ersten Drehachse (140) des Steuerungsübertragungselements (131) ist, wobei die erste Stange (13211) mit dem zweiten Abschnitt (1312) des Steuerungsübertragungselements (131) gekoppelt ist,
- einen ersten Schwenkkopf (13212), der einen Hauptabschnitt (132121) aufweist, der drehbar an der ersten Stange (13211) angebracht ist, und einen ersten Armabschnitt (132122), der sich ab dem Hauptabschnitt (132121) erstreckt, wobei der erste Schwenkkopf (13212) mit dem ersten Bodenstabilisierungsfuß (110) verbunden ist, und
- ein erstes Kugelgelenkende (13213), das drehbar an der ersten Stange (13211) angebracht ist und das drehfest mit der Verbindungsstruktur (1323) verbunden ist.

3. Stabilisierungsvorrichtung (100) nach einem der Ansprüche 1 bis 2, wobei das zweite Gelenk (1322) umfasst
- eine zweite Stange (13221), die eine dritte Drehachse (160) aufweist, die im Wesentlichen parallel zur ersten Drehachse (140) des Steuerungsübertragungselements (131) ist, wobei die zweite Stange (13221) mit dem tragenden Bodenrahmen (210) gekoppelt ist,
- einen zweiten Schwenkkopf (13222), der einen Hauptabschnitt (132221) aufweist, der drehbar an der zweiten Stange (13221) angebracht ist, und einen zweiten Armabschnitt (132222), der sich ab dem Hauptabschnitt (132221) erstreckt, wobei der zweite Schwenkkopf (13222) mit dem zweiten Bodenstabilisierungsfuß (110) verbunden ist, und
- ein zweites Kugelgelenkende (13223), das drehbar an der zweiten Stange angebracht ist und das drehfest mit der Verbindungsstruktur (1323) verbunden ist.

4. Stabilisierungsvorrichtung (100) nach Anspruch 3, wobei das erste Kugelgelenkende (13213) und das zweite Kugelgelenkende (13223) ausgehend von der Verbindungsstruktur (1323) nach oben jeweils bis zu der ersten Stange (13211) und der zweiten Stange (13221) geneigt sind.

5. Stabilisierungsvorrichtung (100) nach einem der Ansprüche 1 bis 4, wobei die Verbindungsstruktur (1323) umfasst
- mindestens einen ersten Endarm (13231), der mit dem ersten Kugelgelenkende (13213) verbunden ist,
- mindestens einen zweiten Endarm (13232), der mit dem zweiten Kugelgelenkende (13223) verbunden ist, und
- einen Mittelkörper (13233), der zwischen dem ersten Kugelgelenkende (13213) und dem zweiten Kugelgelenkende (13223) verbunden ist.

6. Stabilisierungsvorrichtung (100) nach einem der Ansprüche 1 bis 5, wobei der gesamte oder ein Teil des Steuerungsübertragungsmechanismus (130) ausgelegt ist, um abnehmbar angebracht oder dauerhaft fest auf dem tragenden Bodenrahmen (210) befestigt zu sein.

7. Stabilisierungsvorrichtung (100) nach einem der Ansprüche 1 bis 6, wobei der gesamte oder ein Teil des Steuerungsübertragungsmechanismus (130) ausgelegt ist, um abnehmbar angebracht oder dauerhaft fest unter dem tragenden Bodenrahmen (210) befestigt zu sein.

8. Stabilisierungsvorrichtung (100) nach einem der Ansprüche 1 bis 7, wobei die Bodenstabilisierungsfüße (110) in einem Dreieck zwischen einem vorderen Teil und einem hinteren Teil des tragenden Bodenrahmens (210) eingerichtet sind.

9. Stabilisierungsvorrichtung (100) nach einem der Ansprüche 1 bis 8, wobei
- alle oder ein Teil der Bodenstabilisierungsfüße (110) eine Form eines L aufweisen, das in Bezug auf eine Längsachse des tragenden Bodenrahmens (210) geneigt ist, mit jeweils einem langen Schenkel (111) und einem kurzen Schenkel (112), und
- der tragende Bodenrahmen (210) Ausschnitte (211) aufweist, die jeweils eingerichtet sind, um den kurzen Schenkel (112) von mindestens einem Bodenstabilisierungsfuß (110) aufzunehmen, wenn der Bodenstabilisierungsfuß (110) in der eingeklappten Konfiguration ist.

10. Wandernde mobile Roboterstation zur zahnärztlichen oder medizinischen Behandlung, umfassend
- mindestens einen Roboterarm, der ausgelegt ist, um mindestens ein zahnärztliches oder medizinisches Instrument zu bewegen,
- einen medizinischen Wagen (200), der einen tragenden Bodenrahmen (210) umfasst, wobei der tragende Bodenrahmen (210)
- eine Längsachse F hat,
- einen in Bezug auf eine Bewegungsrichtung des medizinischen Wagens (200) vorderen Teil und einen dem vorderen Teil gegenüberliegenden hinteren Teil aufweist, und
- mit Bodenverbindungen (300) versehen ist, die so gestaltet sind, dass er sich auf dem Boden bewegen kann, und
- eine Stabilisierungsvorrichtung (100) nach einem der Ansprüche 1 bis 9.

11. Wandernde mobile Roboterstation zur zahnärztlichen oder medizinischen Behandlung nach Anspruch 10, wobei der gesamte oder ein Teil des Steuerungsübertragungsmechanismus (130) der Stabilisierungsvorrichtung (100) ausgelegt ist, um abnehmbar angebracht oder dauerhaft fest unter dem tragenden Bodenrahmen (210) befestigt zu sein.

12. Wandernde mobile Roboterstation zur zahnärztlichen oder medizinischen Behandlung nach Anspruch 10, wobei der gesamte oder ein Teil des Steuerungsübertragungsmechanismus (130) der Stabilisierungsvorrichtung (100) ausgelegt ist, um abnehmbar angebracht oder dauerhalft fest auf dem tragenden Bodenrahmen (210) befestigt zu sein.

## Claims

1. A stabilization device (100) for a medical cart (200) comprising a carrier floor frame (210), the stabilization device (100) comprising:
- ground stabilizing feet (110) which are configured to support the carrier floor frame (210) and which are convertible between a deployed configuration in which the ground stabilizing feet (110) are bearing on the ground, in a stable manner, so as to support, together, all or part of what the carrier floor frame (210) supports, and a folded configuration in which the ground stabilizing feet (110) are off the ground so as to ensure an at least partial retraction,
- a control mechanism (120) which can be displaced between a first position and a second position, in response to at least one translational movement or one rotational movement, and
- a control transmission mechanism (130) which comprises:
- a connection assembly (132) and
- at least one control transmission element (131) which has a first portion (1311) of said control transmission element (131) kinematically connected, in a direct or indirect manner, to the control mechanism, and a second portion (1312) of said control transmission element (131) kinematically connected, in an articulated manner, to the ground stabilizing feet (110) via the connection assembly (132), the control transmission element (131) being rotatably mounted about a first axis of rotation (140) so as to be driven in rotation in a first direction of rotation and in a second direction of rotation opposite to the first direction of rotation,
said connection assembly (132)comprising,
- at least a first articulation (1321), with at least one degree of freedom, which is directly connected to the second portion (1312) of said control transmission element (131) and to at least a first ground stabilizing foot (110),
- at least a second articulation (1322), with at least one degree of freedom, which is directly connected to at least a second ground stabilizing foot (110), and
- a connection structure (1323) which is connected between the first articulation (1321) and the second articulation (1322),
wherein,
the control transmission mechanism (130) is configured so that the actuation of the control mechanism (120) in the first configuration or in the second configuration drives in rotation the control transmission element (131) about the first axis of rotation, and allows the ground stabilizing feet (110) to alternate between the deployed configuration and the folded configuration.

2. The stabilization device (100) according to claim 1, wherein the first articulation (1321) comprises:
- a first rod (13211) which has a second axis of rotation (150) which is substantially parallel to the first axis of rotation (140) of the control transmission element (131), the first rod (13211) being coupled to the second portion (1312) of said control transmission element (131),
- a first pivoting head (13212) which has a main section (132121) which is mounted in rotation on the first rod (13211) and a first arm section (132122) which extends from the main section (132121), the first pivoting head (13212) being connected to the first ground stabilizing foot (110), and
- a first ball-joint tip (13213) which is mounted in rotation on the first rod (13211) and which is connected, immobilized in rotation, to the connection structure 1323.

3. The stabilization device (100) according to any one of claims 1 to 2, wherein the second articulation (1322) comprises:
- a second rod (13221) which has a third axis of rotation (160) which is substantially parallel to the first axis of rotation (140) of the control transmission element (131), the second rod (13221) being coupled to the carrier floor frame (210),
- a second pivoting head (13222) which has a main section (132221) which is mounted in rotation on the second rod (13221) and a second arm section (132222) which extends from the main section (132221), the second pivoting head (13222) being connected to the second ground stabilizing foot (110), and
- a second ball-joint tip (13223) which is mounted in rotation on the second rod and which is connected, immobilized in rotation, to the connection structure (1323).

4. The stabilization device (100) according to claim 3, wherein the first ball-joint tip (13213) and the second ball-joint tip (13223) are inclined upwards from the connection structure (1323) to, respectively, the first rod (13211) and the second rod (13221).

5. The stabilization device (100) according to any one of claims 1 to 4, wherein the connection structure (1323) comprises:
- at least a first end arm (13231) which is connected to the first ball-joint tip (13213),
- at least a second end arm (13232) which is connected to the second ball-joint tip (13223), and
- a central body (13233) which is connected between the first ball-joint tip (13213) and the second ball-joint tip (13223).

6. The stabilization device (100) according to any one of claims 1 to 5, wherein all or part of the control transmission mechanism (130) is configured to be removably mounted or permanently fixed over the carrier floor frame (210).

7. The stabilization device (100) according to any one of claims 1 to 6, wherein all or part of the control transmission mechanism (130) is configured to be removably mounted or permanently fixed under the carrier floor frame (210).

8. The stabilization device (100) according to any one of claims 1 to 7, wherein the ground stabilizing feet (110) are arranged in a triangle between a front part and a rear part of the carrier floor frame (210).

9. The stabilization device (100) according to any one of claims 1 to 8, wherein,
- all or part of the ground stabilizing feet (110) have an L shape inclined relative to a longitudinal axis of the carrier floor frame (210), each with a long branch (111) and a short branch (112), and
- the carrier floor frame (210) has cutouts (211), each being arranged to receive the short branch (112) of at least one ground stabilizing foot (110), when the ground stabilizing foot (110) is in the folded configuration.

10. A movable itinerant robotic dental or medical treatment station comprising:
- at least one robotic arm which is configured to move at least one dental or medical instrument,
- a medical cart (200) which comprises a carrier floor frame (210), the carrier floor frame (210),
- having a longitudinal axis, F,
- having a front part relative to a direction of displacement of the medical cart (200) and a rear part opposite to the front part, and
- being provided with ground connections (300) designed to allow it to move on the ground, and
- a stabilization device (100) according to any one of claims 1 to 9.

11. The movable itinerant robotic dental or medical treatment station according to claim 10, wherein all or part of the control transmission mechanism (130) of the stabilization device (100) is configured to be removably mounted or permanently fixed under the carrier floor frame (210).

12. The movable itinerant robotic dental or medical treatment station according to claim 10, wherein all or part of the control transmission mechanism (130) of the stabilization device (100) is configured to be removably mounted or permanently fixed over the carrier floor frame (210).
